# FASCICULE DE BREVET EUROPEEN

(11) **EP 1 275 372 B1**
(45) Date de publication et mention de la délivrance du brevet: **13.06.2007**
(21) Numéro de dépôt: 02291597.9
(22) Date de dépôt: 26.06.2002
(51) Int. Cl.: A61K 8/31, A61K 8/44, A61K 8/49, A61K 8/67, A61Q 7/00, A61Q 19/00, A61Q 19/08

(54) **Composition cosmétique ou dermatologique comprenant un dérivé de N-Acylaminoamide et un inhibiteur de métalloprotéinase**
Kosmetische und dermatologische Zusammensetzung enthaltend ein N-Acylaminoamid-Derivat und einen Metalloproteinase-Inhibitor
Cosmetic or dermatological composition comprising a N-acylaminoamide derivative and a metalloproteinase inhibitor

(30) Priorité: 26.06.2001 FR 0108433
(43) Date de publication de la demande: 15.01.2003
(73) Titulaire: L'OREAL, S.A., 75008 Paris (FR)
(72) Inventeur: Breton, Lionel, 7800 Versailles (FR); Mahe, Yann, 91390 Morsang sur Orge (FR)
(74) Mandataire: Catherine, Alain

(56) Documents cités:
- EP-A- 0 985 409
- EP-A- 1 000 613
- EP-A- 1 090 628
- WO-A-00/12467
- WO-A-98/36742
- FR-A- 2 810 033
- US-A- 5 234 909
- NAKAMURA M ET AL: "A two-step, one-pot synthesis of diverse N-pyruvoyl amino acid derivatives using the Ugi reaction" BIOORGANIC & MEDICINAL CHEMISTRY LETTERS, OXFORD, GB, vol. 10, no. 24, 18 décembre 2000 (2000-12-18), pages 2807-2810, XP004225356 ISSN: 0960-894X

## Description

La présente invention se rapporte au domaine des compositions cosmétiques ou dermatologiques. Elle est relative à de nouvelles compositions cosmétiques ou dermatologiques comprenant une association entre un composé inhibiteur de l'élastase de la famille des N-Acylaminoamides et au moins un antagoniste de la synthèse et/ou de la libération et/ou de l'activité de métalloprotéinases matricielles. Cette composition est préférentiellement destinée a améliorer les signes cutanés du vieillissement et/ou du photovieillissement -signes cutanés qui, pour certains d'entre eux, sont directement la résultante d'un processus micro-inflammatoire chronique induit par des expositions répétées aux UVs.

La peau humaine est constituée de deux compartiments à savoir un compartiment superficiel, l'épiderme, et un compartiment profond, le derme. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il est également traversé par des vaisseaux sanguins et des fibres nerveuses.

On sait que lors d'un stress cutané superficiel, qui peut notamment être d'origine chimique, physique ou bactérienne, les kératinocytes des couches superficielles de l'épiderme libèrent des médiateurs biologiques qui possèdent la capacité d'attirer certaines cellules infiltrantes de la peau, elles-mêmes responsables de l'entretien d'une irritation locale transitoire.

Parmi les médiateurs biologiques pouvant être produits par les kératinocytes ainsi stressés, on citera les chimiokines qui sont des cytokines chimioattractives responsables du recrutement de leucocytes sur les sites inflammatoires, dont l'interleukine 8 (IL-8) qui est plus particulièrement responsable du recrutement des neutrophiles.

Ces cellules infiltrant les zones irritées ou agressées libèrent alors des enzymes parmi lesquelles on peut citer l'élastase leucocytaire.

Sous l'action de cette enzyme notamment, les fibres élastiques de soutien extracellulaire du tissu conjonctif peuvent être dégradées, et entraîner ainsi une diminution de l'élasticité de la peau.

Il est même par ailleurs connu qu'en synergie avec la cathepsine G, l'élastase leucocytaire peut dissocier l'intégrité de l'épiderme en élargissant les espaces intercellulaires interkératinocytaires.

Ainsi, à long terme, la somme des micro-stress cutanés superficiels, par exemple générés par une exposition prolongée aux UV ou par des agents irritants, peut entraîner une perte plus ou moins accélérée de l'élasticité naturelle de la peau. Le réseau formé par les fibres élastiques du tissu conjonctif sous-jacent et des espaces extracellulaires peut alors progressivement être destructuré. Il s'en suit un vieillissement accéléré de la peau (peau ridée et/ou moins souple) via l'altération du réseau élastique dermique, ainsi qu'une accentuation des rides (rides plus profondes).

Par ailleurs, on sait que la solidité du derme est également assurée par les fibres de collagène. Ces fibres sont constituées de fibrilles scellées les unes aux autres, formant ainsi plus de dix types de structures différentes. La solidité du derme est également due à l'enchevêtrement des fibres de collagène tassées les unes contre les autres en tous sens. Les fibres de collagène participent à l'élasticité et à la tonicité de la peau et/ou des muqueuses.

Les fibres de collagènes sont constamment renouvelées mais ce renouvellement diminue avec l'âge ce qui entraîne également un amincissement du derme. Cet amincissement du derme est également dû à des causes pathologiques comme par exemple l'hypersécrétion d'hormones corticoïdes, certaines pathologies ou encore des carences vitaminiques (cas de la vitamine C dans le scorbut). Il est également admis que des facteurs extrinsèques comme les rayons ultraviolets, le tabac ou certains traitements (Glucocorticoïdes, vitamine D et dérivés par exemple) ont également un effet sur la peau et sur son taux de protéines matricielles, particulièrement de collagène.

Bien que très résistantes, les fibres de collagène sont sensibles à certaines enzymes appelées collagénases. Une dégradation des fibres de collagène entraîne l'apparence de peau molle et ridée que l'être humain, préférant l'apparence d'une peau lisse et tendue, cherche depuis toujours à combattre.

Par ailleurs à la ménopause, les principales modifications concernant le derme sont une altération du tissu élastique et une diminution du taux de collagène et de l'épaisseur dermique. Cela entraîne chez la femme ménopausée un amincissement de la peau et/ou des muqueuses. La femme ressent alors une sensation de "peau sèche" ou de peau qui tire et l'on constate une accentuation des fines rides et ridules de surface. La peau présente un aspect rugueux à la palpation. Enfin la peau présente une souplesse diminuée.

Lors d'un stress cutané (chimique, physique, bactérien ou neurogène) les kératinocytes libèrent des médiateurs biologiques (appelés facteurs chimioattractants) qui possèdent la capacité d'attirer certaines cellules inflammatoires du compartiment sanguin vers le tissu cutané . Ces cellules sont responsables de la genèse puis de l'entretien d'une irritation locale.

Parmi les facteurs chimio-attractants pouvant être produits par les kératinocytes stressés, l'interleukine 8 (IL-8) est plus spécifiquement responsables du recrutement des polynucléaires neutrophiles. Ces cellules infiltrant les zones irritées ou agressées libèrent alors des enzymes parmi lesquelles l' élastase leucocytaire et d'autres protéases (metalloprotéinases, serines protéases etc...)

Sous l'action de cette enzyme, les fibres élastiques de soutien extracellulaire du tissu conjonctif sont dégradées. En synergie avec la cathepsine G, l'élastase leucocytaire peut également dissocier l'intégrité de l'épiderme en élargissant les espaces intercellulaires inter-kératinocytaires (Ludolph-Hauser et al Exp. Dermatol. 1999 8(1) 46-52). L'élastase leucocytaire a récemment été incriminée dans l'entretien des escarres et la survenue des ulcères veineux des jambes, via son activité de dégradation de la fibronectine (Herrick S et al Lab Invest. 1997(3) 281-288. La somme des micro-stress localisés de dégradation (suite par exemple à une exposition prolongée au soleil) peut aboutir au long terme à une perte accélérée de l'élasticité naturelle de la peau. Le réseau des fibres élastiques du tissu conjonctif sous jacent et des espaces extracellulaires est alors progressivement déstructuré. Cette dégradation accélérée peut se cumuler avec le processus de vieillissement normal de la peau qui se caractérise par une plus grande sensibilité des fibres élastiques à l'action de l'élastase (Stadler R & Orfanos CE Arch. Dermatol. Res. 1978 262 (1) 97-111

Il est connu dans l'état de la technique d'apporter, dans le tissu cutané, des molécules capables de ralentir l'activité de dégradation des fibres élastiques des espaces intercellulaires.

Or, ceci n'est pas toujours suffisant. En effet, le réseau fibrillaire est complexe et de nombreuses autres activités enzymatiques peuvent aussi dégrader l' Elastine et le collagène et, par voie de conséquence déstructurer et désorganiser, par un site distinct de l' élastine, les mailles du réseau cutané collagéno-élastique. Parmi ces enzymes, citons les metalloprotéinases et gélatinases capables de dégrader le collagène natif (comme la MMP-1, la MMP-2 et la MMP-14) ou dénaturé sous forme de gélatine (comme la MMP-9 et la MMP-2).

La solution technique selon l'invention consiste à apporter, en plus de l'élément régulateur de l'activité élastasique (le dérivé N-Acylaminoamide, inhibiteur de l'activité enzymatique de l' élastase leucocytaire) un ou plusieurs actifs capables de réguler aussi les autres activités enzymatiques qui interfèrent avec l'intégrité du réseau matriciel cutané, sous la forme de compositions.

Cette nouvelle association peut être utilisée dans des préparations cosmétiques de soin des zones exposées au soleil (scalp, corps, visage, lèvres), dans les préparation cosmétiques de soin des zones ulcérées, dans les dentifrices ou lotions de rinçage buccales et, de façon générale, dans toutes les préparations cosmétiques dites " anti-vieillissement cutané " qui ont pour objectif de ralentir la déstructuration chronobiologique des tissus de soutien et de l'architecture des éléments matriciels cutanés .

En conséquence, l'invention a pour objet une composition cosmétique ou dermatologique caractérisée en ce qu'elle comprend une association entre un composé inhibiteur de l'élastase de la famille des N-Acylaminoamides et au moins un inhibiteur de métalloprotéinase.

Par " inhibiteur de métalloprotéinase " au sens de l'invention, on entend un composé antagoniste de la synthèse et/ou de la libération et/ou de l'activité des métalloprotéinases matricielles.

Un autre objet de l'invention consiste en un procédé de traitement cosmétique de la peau du corps ou du visage, y compris le cuir chevelu, dans lequel on applique sur la peau une composition cosmétique telle que définie ci-dessus.

Il a en effet été constaté que les composés de formule (I) présentaient une activité inhibitrice de l'activité des élastases, et qu'ils pouvaient donc être employés pour limiter et/ou combattre la dégradation des fibres élastiques. Il s'en suit qu'ils peuvent être employés dans ou pour la préparation d'une composition, les composés ou la composition étant destinés à traiter, de manière préventive et/ou curative, les signes cutanés du vieillissement.

L'association nouvelle des composés N-Acylaminoamides avec au moins un inhibiteur de métalloprotéinase permet de renforcer significativement l'effet antivieillissement du tissu matriciel, par l'apport d'un effet à la fois sur des enzymes impliquées dans la dégradation de l'élastine et sur des enzymes impliquées dans la dégradation du collagène.

Selon l'invention, l'élément régulateur de l'activité élastasique (i.e le dérivé N-Acylaminoamide inhibiteur de l'activité enzymatique de l'élastase leucocytaire, l'acide {2-[acétyl-(3-trifluorométhyl-phényl)-amino]-3-méthyl-butyrylamino} acétique) est combiné à un ou plusieurs actifs capables d'inhiber l'activité, la synthèse ou la libération des métalloprotéinases de la peau.

La composition obtenue est destinée à traiter les désordres du vieillissement et/ou à être plus spécifiquement destinée à traiter tous les signes cutanés du vieillissement et/ou du photovieillissement.

Préférentiellement cette nouvelle association est utilisée dans des préparations cosmétiques de soin des zones exposées au soleil (scalp, corps, visage, lèvres) et, de façon générale, dans toutes les préparations cosmétiques dites " anti-vieillissement cutané " qui ont pour objectif de ralentir la destructuration des tissus de soutien et de l'architecture des éléments matriciels cutanés .

Sans vouloir être lié par une quelconque théorie, le demandeur considère que le fait d'apporter, au niveau des kératinocytes des couches superficielles de la peau, des composés susceptibles de ralentir l'activité de dégradation des fibres élastiques des espaces intercellulaires, peut permettre de diminuer ce phénomène de vieillissement accéléré de la peau, dû à des stress cutanés superficiels et que l'association de ces composés avec un inhibiteur de métalloprotéinase renforce considérablement leurs effets. Le document US-A-5 234 909 décrit des compositions, contenant des amides dipeptidiques dérivés de glycyl-sérine, pour la traitement et le soin de la peau.

### Composés N-acylamino-amides

Les composés employés dans la présente invention répondent donc à la formule (I) suivante : 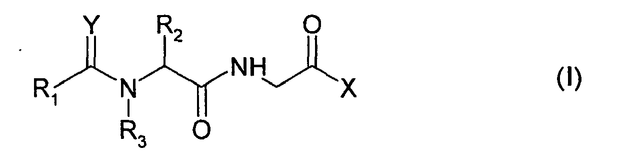 dans laquelle:
- le radical Y représente O,
- le radical R1 représente un radical méthyle, éthyle, propyle ou isopropyle, éventuellement substitué par un groupement -OH ou - P(O)-(OR)₂ avec R représentant méthyle, éthyle, propyle ou isopropyle,
- le radical R2 représente un radical méthyle, éthyle, propyle, isopropyle, n-butyle, ter-butyle ou isobutyle,
- le radical R3 représente un groupement choisi parmi l'une des formules suivantes : 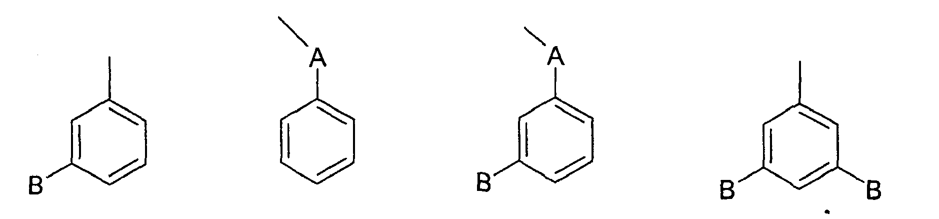
dans lesquelles le radical divalent A est un méthylène, un éthylène, un propylène,

le radical B est un radical méthyle, éthyle, propyle ou isopropyle, substitué par un ou plusieurs halogènes, notamment chlore, brome, iode ou fluor, et préférentiellement totalement halogéné (perhalogéné), tel que perfluoré; on peut en particulier citer le radical perfluorométhyle (-CF3) comme tout particulièrement préféré,
- le radical X représente un radical choisi parmi -OH, - OCH₃, -OC₂H₅, -O-C₃H₇ ou -OC₄H₉, ses sels d'acide minéral ou organique, ses isomères optiques, sous forme isolée ou en mélange racémique.

Parmi les composés particulièrement préférés, on peut citer :
- l'acide {2-[acétyl-(3-trifluorométhyl-phényl)-amino]-3-méthyl-butyrylamino} acétique,
- le {2-[acétyl-(3-trifluorométhyl-phényl)-amino]-3-méthyl-butyrylamino} acétate d'éthyle,
- l'acide [2-(acétyl-benzyl-amino)-3-méthyl-butyrylamino] acétique,
- le [2-(acétyl-benzyl-amino)-3-méthyl-butyrylamino] acétate d'éthyle,
- le (2-{benzyl-[(diethoxy-phosphoryl)-acétyl]-amino}-3-méthyl-butyrylamino) acétate d'éthyle.

Les composés selon l'invention peuvent être aisément préparés par l'homme du métier sur base de ses connaissances générales. On peut notamment faire réagir ensemble un acide carboxylique, un aldéhyde, un composé aminé et un isonitrile, selon la réaction de Ugi.

Bien entendu, lors de la synthèse des composés selon l'invention, et en fonction de la nature des différents radicaux présents sur les composés de départ, l'homme du métier pourra veiller à protéger certains substituants pour que ceux-ci n'interviennent pas dans la suite des réactions.

La quantité de composé à utiliser dans les compositions selon l'invention peut être aisément déterminée par l'homme du métier, en fonction de la nature du composé utilisé, de la personne à traiter et/ou de l'effet recherché. D'une manière générale, cette quantité peut être comprise entre 0,00001 et 20% en poids par rapport au poids total de la composition, et préférentiellement entre 0,0001 et 5 % en poids.

Les composés de formule (I) peuvent notamment être employés, dans une composition qui comprend un milieu physiologiquement acceptable, notamment dans une composition cosmétique ou pharmaceutique qui comprend donc par ailleurs un milieu cosmétiquement ou pharmaceutiquement acceptable.

Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

D'une manière générale, ce milieu peut être anhydre ou aqueux. Il peut ainsi comprendre une phase aqueuse et/ou une phase grasse.

### Inhibiteurs de métalloprotéinases préférés

Par "inhibiteur de métalloprotéinase", on entend selon l'invention, toute molécule et/ou extrait végétal ou bactérien présentant une activité inhibitrice sur l'activité, la synthèse ou la libération des métalloprotéinases de la peau.

Les métalloprotéinases sont notamment décrites dans l'article de Y. HEROUY et al., European Journal of Dermatology, n° 3, vol. 10, Avril-Mai 2000, pp. 173-180.

La famille des métalloprotéinases est ainsi constituée de plusieurs groupes bien définis basés sur leurs ressemblances en terme de structure et de spécificité de substrat (voir Woessner J. F., Faseb Journal, vol. 5, 1991, 2145). Parmi ces groupes, on peut citer les collagénases destinées à dégrader les collagènes fibrillaires (MMP-1 ou collagénase interstitielle, MMP-8 ou collagénase de neutrophile, MMP-13 ou collagénase 3, MMP-18 ou collagénase 4), les gélatinases qui dégradent le collagène de type IV ou toute forme de collagène dénaturé (MMP-2 ou gélatinase A (72 kDa), MMP-9 ou gélatinase B (92 kDa)), les stromélysines (MMP-3 ou stromélysine 1, MMP-10 ou stromélysine 2, MMP-11 ou stromélysine 3) dont le large spectre d'activité s'adresse aux protéines de la matrice extracellulaire telles que les glycoprotéines (fibronectine, laminine), les protéoglycannes, etc., la matrilysine (MMP-7), la métalloélastase (MMP-12) ou encore les métalloprotéinases membranaires (MMP-14, MMP-15, MMP-16 et MMP-17).

Les métalloprotéinases (MMPs) sont les membres d'une famille d'enzymes protéolytiques (endoprotéases) qui possèdent un atome de zinc coordonné à 3 résidus cystéine et à une méthionine dans leur site actif et qui dégradent les composants macromoléculaires de la matrice extracellulaire et des lames basales à pH neutre (collagène, élastine, etc ...). Très largement répandues dans le monde vivant, ces enzymes sont présentes, mais faiblement exprimées, dans des situations physiologiques normales comme la croissance des organes et le renouvellement des tissus. Leur surexpression chez l'homme et leur activation sont cependant liées à de nombreux processus qui impliquent la destruction et le remodelage de la matrice. Cela entraîne par exemple une résorption non contrôlée de la matrice extracellulaire.

Ainsi, l'exposition prolongée aux rayonnements ultraviolets, particulièrement aux rayonnements ultraviolets de type A et/ou B, a pour effet une stimulation de l'expression des collagénases, particulièrement de la MMP-1. C'est là une des composantes du vieillissement cutané photo-induit. En outre, on sait que l'activité des MMP-1, MMP-2 et MMP-9 augmente avec l'âge et que cette augmentation contribue, avec le ralentissement de la croissance cellulaire, au vieillissement chronologique de la peau (WO 98/36742).

Les métalloprotéinases sont produites et sécrétées sous une forme inactive (pro-enzyme). Ces formes inactives dites zymogènes sont ensuite activées dans l'environnement extracellulaire par l'élimination d'une région propeptidique. Les membres de cette famille peuvent .s'activer les uns les autres. La régulation de l'activité des MMPs se produit ainsi au niveau de l'expression des gènes (transcription et traduction), au niveau de l'activation de la forme zymogène, ou au niveau du contrôle local des formes actives.

Les régulateurs naturels de l'activité des MMPs sont les inhibiteurs tissulaires des métalloprotéinases ou TIMPs (tissue inhibitors of metalloproteinases). Cependant, l'expression des MMPs est également modulée par les facteurs de croissance, les cytokines, les produits oncogènes (ras, jun), ou encore les constituants matriciels.

Par inhibiteur de métalloprotéinase selon l'invention, on entend toute molécule capable de réguler l'activité des MMPs soit au niveau de l'expression des gènes (transcription et traduction), soit au niveau de l'activation de la forme zymogène des MMPs, soit encore au niveau du contrôle local des formes actives.

Préférentiellement, une composition selon l'invention est caractérisée en ce que l'inhibiteur de métalloprotéinase est choisi parmi un inhibiteur de métalloprotéinase choisie parmi les MMP-1, MMP-2, MMP-3, MMP-7, MMP-9, MMP-11, MMP-12, MMP-13, MMP-14, MMP-15, MMP-16 et MMP-17.
L'inhibiteur de métalloprotéinase selon l'invention peut être un inhibiteur naturel de métalloprotéinases, particulièrement un inhibiteur tissulaire des métalloprotéinases (TIMP) tel que les peptides connus dans l'art antérieur sous les appellations TIMP-1, TIMP-2, TIMP-3 et TIMP-4 (Woessner J. F., Faseb Journal, 1991).

En variante, les inhibiteurs de métalloprotéinases convenant à une mise en oeuvre dans la présente invention peuvent être des inhibiteurs de MMP-1 d'origine naturelle ou synthétique. Par "origine naturelle", on entend l'inhibiteur de métalloprotéinase à l'état pur ou en solution à différentes concentrations, obtenu par différents procédés d'extraction à partir d'un élément, généralement une plante, d'origine naturelle. Par "origine synthétique", on entend l'inhibiteur de métalloprotéinase à l'état pur ou en solution à différentes concentrations, obtenu par synthèse chimique.

L'inhibiteur de métalloprotéinase peut aussi être un extrait naturel contenant de l'acide Ursolique ou des caroténoïdes ou de la vitamine C ou les isoflavones comme la genistéine connue pour son activité inhibitrice de métalloprotéinase (US6130254).

Selon une forme d'exécution préférée de l'invention, on utilise un inhibiteur de métalloprotéinase d'origine naturelle, tel que le lycopène ou une isoflavone. L'activité du lycopène sur les métalloprotéinases a été décrite dans la demande de brevet EP-A-1090628.

Selon un autre mode de réalisation préféré, l'inhibiteur de métalloprotéinase est un inhibiteur transcriptionnel de la métalloprotéinase MMP-1, tel que le rétinol ou ses dérivés, l'acide rétinoïque et ses dérivés.

Les inhibiteurs à associer pourront également être choisis parmi l'acide retinoïque ou ses dérivés, l'adapalène ou encore des peptides analogues et/ou des dérivés du Batimastat ((BB 94 ) =[4-(N-hydroxyamino)-2R-isobutyl-3S-(thiophen-2-ylthiomethyl)-succinyl]-L-phenylalanine-N-methylamide), du Marimastat ((BB 2516)=[2S-[N4(R*), 2R*,3S]]-N4[2,2-dimethyl-1-[(methylamino)carbonyl]propyl]-N1,2-dihydroxy-3-(2-methylpropyl)butanediamide) vendus par la société British Biotech ou encore des inhibiteurs biologiques naturels comme les inhibiteurs tissulaires des metalloproteinases (TIMPs), ainsi que des analogues structuraux et /ou fonctionnels voire même des inducteurs de la synthèse et/ou de la libération de ces inhibiteurs naturels.

Les filtres solaires, par une action indirecte sur la transcription et/ou la synthèse de métalloprotéinases, sont également utilisables selon l'invention.

Plus préférentiellement, une composition selon l'invention contiendra du rétinol en tant qu'inhibiteur de métalloprotéinase.

Idéalement, cette nouvelle association pourra être utilisée dans des préparations cosmétiques de soin des zones exposées au soleil (scalp, corps, visage, lèvres) et, de façon générale, dans toutes les préparations cosmétiques dites " anti-vieillissement cutané " qui ont pour objectif de ralentir la destructuration chronobiologique des tissus de soutien et de l'architecture des éléments matriciels cutanés.

L'inhibiteur de métalloprotéinase représente de préférence de 10⁻¹² à 5%, et de préférence de 10⁻¹⁰ à 2%, du poids total de la composition. Bien entendu, si l'inhibiteur de métalloprotéinase est présent sous forme de solution contenant un extrait de plante, l'homme du métier saura ajuster la quantité de cette solution dans la composition selon l'invention, de façon à obtenir l'effet attendu sur l'activité et/ou la synthèse et/ou la libération des métalloprotéinases.

L'association d'au moins un composé N-acylamino-amide et d'au moins un inhibiteur de métalloprotéinase peut notamment être employée, seule ou en mélange, dans une composition qui comprend un milieu physiologiquement acceptable, notamment dans une composition cosmétique ou pharmaceutique qui comprend donc par ailleurs un milieu cosmétiquement ou pharmaceutiquement acceptable.

Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

D'une manière générale, ce milieu peut être anhydre ou aqueux. Il peut ainsi comprendre une phase aqueuse et/ou une phase grasse.

Pour une application sur la peau, la composition peut avoir la forme notamment de solution aqueuse ou huileuse; de dispersion du type lotion ou sérum; d'émulsions de consistance liquide ou semi-liquide du type lait obtenues par dispersion d'une phase grasse dans une phase aqueuse (H/E) ou inversement (E/H); de suspensions ou émulsions de consistance molle du type crème ou gel aqueux ou anhydres; de microcapsules ou microparticules; de dispersions vésiculaires de type ionique et/ou non ionique.

Pour une application sur les cheveux, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.

Ladite phase aqueuse peut comprendre en outre des alcools tels que des monoalcools en C₁-C₆ et/ou des polyols tels que le glycérol, le butylèneglycol, l'isoprène glycol, le propylèneglycol, le polyéthylèneglycol.

Lorsque la composition selon l'invention se présente sous la forme d'une émulsion, elle peut éventuellement comprendre en outre un tensioactif, de préférence en une quantité de 0,01 à 30% en poids par rapport au poids total de la composition. La composition selon l'invention peut également comprendre au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut également comprendre une phase grasse, notamment constituée de corps gras liquides à 25°C, tels que des huiles d'origine animale, végétale, minérale ou synthétique, volatiles ou non; de corps gras solides à 25°C tels que des cires d'origine animale, végétale, minérale ou synthétique; de corps gras pâteux; de gommes; de leurs mélanges.

Les huiles volatiles sont généralement des huiles ayant, à 25°C, une tension de vapeur saturante au moins égale à 0,5 millibar (soit 50 Pa).

Parmi les constituants de la phase grasse, on peut citer :
- les silicones volatiles cycliques ayant de 3 à 8 atomes de silicium, de préférence de 4 à 6.
- les cyclocopolymères du type diméthylsiloxane/méthylalkylsiloxane,
- les silicones volatiles linéaires ayant de 2 à 9 atomes de silicium.
- les huiles volatiles hydrocarbonées, telles que les isoparaffines et notamment l'isododécane et des huiles fluorées.
- les polyalkyl(C₁-C₂₀) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule R1COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.
- les gommes de silicones;
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

De façon connue, la composition selon l'invention peut comprendre les adjuvants habituels dans le domaine considéré, tels que les gélifiants hydrophiles ou lipophiles, les additifs hydrophiles ou lipophiles, les actifs notamment cosmétiques ou pharmaceutiques hydrophiles ou lipophiles, les conservateurs, les antioxydants, les solvants, les parfums, les charges, les pigments, les nacres, les filtres UV, les absorbeurs d'odeur et les colorants. Ces adjuvants, selon leur nature, peuvent être introduits dans la phase grasse, dans la phase aqueuse et/ou dans des sphérules lipidiques.

La nature et la quantité de ces adjuvants peuvent être choisies par l'homme du métier, sur la base de ses connaissances générales, de manière à obtenir la forme de présentation désirée pour la composition. En tout état de cause, l'homme du métier veillera à choisir tous les éventuels composés complémentaires et/ou leur quantité, de manière telle que les propriétés avantageuses de la composition selon l'invention ne soient pas, ou substantiellement pas, altérées par l'adjonction envisagée.

Les compositions cosmétiques ou pharmaceutiques selon l'invention peuvent notamment se présenter sous la forme d'une composition destinée au soin et/ou au traitement des zones ulcérées ou ayant subi un stress ou microstress cutané, notamment généré par une exposition aux UV et/ou la mise en contact avec un produit irritant.

Ainsi, les compositions selon l'invention peuvent notamment se présenter sous la forme :
- d'un produit de soin, de traitement, de nettoyage ou de protection, de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées; une composition démaquillante; un lait pour le corps, notamment hydratant éventuellement après-soleil;
- d'une composition de protection solaire, de bronzage artificiel (autobronzant) ou de soin après-soleil;
- d'une composition capillaire, et notamment une crème ou un gel protecteur solaire; une composition de soin du cuir chevelu, notamment anti-chute ou repousse des cheveux; un shampooing antiparasitaire;
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres;
- d'un produit d'hygiène buccale tel qu'une pâte dentifrice ou une lotion de rinçage buccal.

Les compositions selon l'invention trouvent une application préférée comme composition de soin de la peau du visage, de type anti-rides ou anti-age, et comme composition de protection solaire ou après-soleil.

La présente invention a également pour objet un procédé de traitement cosmétique de la peau du corps ou du visage, y compris le cuir chevelu, dans lequel on applique sur la peau une composition cosmétique comprenant une association entre un composé de la famille des N-acylamino-amides et au moins un inhibiteur de métalloprotéinase, à laisser celle-ci en contact puis éventuellement à rincer.

Le procédé de traitement cosmétique de l'invention peut être mis en oeuvre notamment en appliquant les compositions cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs; application d'une lotion pour cuir chevelu sur cheveux mouillés; application de dentifrice sur les gencives.

L'invention est illustrée plus en détail dans les exemples suivants.

### Exemple 1

### Préparation du {2-[acétyl-(3-trifluorométhyl-phényl)-amino]-3-méthyl-butyrylamino} acétate d'éthyle de formule :

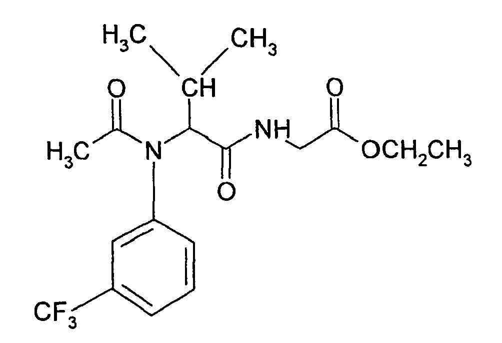

On mélange 0,63 ml d'isobutyraldéhyde et 1 ml de triftuorométhylphénylamine (1,15 eq) dans 15 ml de méthanol, sous agitation. On laisse réagir 15 minutes à 20°C, puis on ajoute 0,46 ml d'acide acétique (1,15 eq) et on laisse réagir 10 minutes à 20°C. On ajoute alors 0,8 ml d'isocyanoacétate d'éthyle à 95% (1 eq) et on laisse réagir 48 heures à 20°C.

On concentre le milieu réactionnel au rotovapor et on purifie le résidu sur colonne de silice (éluant : heptane : 3 / acétate d'éthyle : 7; Rf = 0,5).

On obtient 2,45 g de composé sous forme de solide cireux, d'où un rendement de 91 %.
RMN ¹H (200 MHz; CDCl3) δ ppm : 0,9 (6H;q), 1,3 (3H;t), 1,8 (3H;s), 2,3 (1H;m), 4,0 (2H,q), 4,2 (2H;q), 4,4 (2H;d), 7,3 (1 H;t), 7,5 (4H;m)

### Exemple 2

Préparation de l'acide {2-[acétyl-(3-trifluorométhyl-phényl)-amino]-3-méthyl-butyrylamino} acétique de formule :

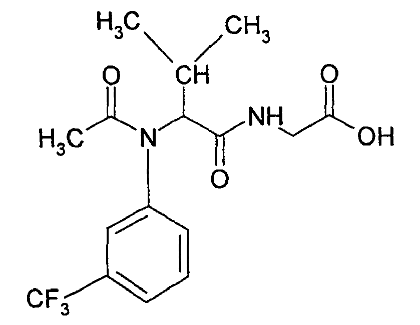

On solubilise 2 g de composé préparé à l'exemple 1 dans 30 ml d'acétone. On ajoute 30 ml de soude 2N et on laisse réagir pendant 6 heures à 20°C. On concentre le milieu réactionnel au rotovapor. On acidifie la phase aqueuse résiduelle à pH 2 par ajout d'HCl concentré puis on extrait par CH₂Cl₂.

La phase organique est concentrée à sec après séchage sur sulfate de sodium.

On obtient un résidu qui est solubilisé par un mélange eau basique à 10% d'éthanol, puis de nouveau acidifié par HCl concentré à pH 2. On extrait une nouvelle fois par Ch2Cl2 et on sèche la phase organique sur sulfate de sodium, on la filtre et on la concentre à sec sous vide au rotovapor.

On obtient 1,3 g de composé sous forme d'un solide légèrement marron clair, d'où un rendement de 70%.
RMN ¹H (200 MHz; DMSO) δ ppm : 0,9 (6H;q), 3,7 (2H;m), 1,8 (4H;m), 4,8 (2H;d), 7,6 (4H,m),8,4 (1 H;t), 12,5 (1 H;s)

### Exemple 3

On a déterminé in vitro l'activité anti-élastasique de composés selon l'invention, vis-à-vis de l'élastase leucocytaire humaine (ELH).

Le test est effectué de la manière suivante :
On laisse incuber, à 37°C, pendant 60 minutes, un substrat Me-OSAAPV-p-NA (Méthyl-O-succinate alanine alanine proline valine-p-nitroanilide) sur lequel est appliqué de l'ELH (40 milli-unités par ml) et 0,1% du composé à tester.
On détermine ensuite par spectrophotométrie le % d'inhibition de l'activité élastase témoin.
Les composés testés sont les suivants :
   Composé A : acide {2-[acétyt-(3-trifiuorométhyl-phényl)-amino]-3-méthyl-butyryl-amino} acétique
   Composé B : (2-{benzyl-[(diethoxy-phosphoryl)-acétyl]-amino}-3-méthyl-butyryl-amino) acétate d'éthyle
   Composé C : acide [2-(acétyl-benzyl-amino)-3-méthyl-butyrylamino] acétique
   Composé D : [2-(acétyl-benzyl-amino)-3-méthyl-butyrylamino] acétate d'éthyle

On obtient les résultats suivants :

| Composé (concentration : 0,1%) | % d'inhibition de l'activité élastase témoin |
|---|---|
| Composé A | 67% |
| Composé B | 17% |
| Composé C | 20% |
| Composé D | 13% |

On détermine de la même manière le % d'inhibition de l'activité élastase témoin pour le composé A, à différentes concentrations.

On obtient les résultas suivants :

| Concentration du composé A | % d'inhibition de l'activité élastase témoin |
|---|---|
| 0,01 % | 53% |
| 0,05% | 50% |
| 0,1% | 68% |
| 0,2% | 68% |

Le composé A provoque donc une forte inhibition de l'activité élastase, même en une quantité faible.

### Exemple 4

On a évalué l'activité ex vivo du composé de l'exemple 2 sur des peaux humaines en survie traitées par de l'élastase leucocytaire humaine (ELH).

Le test est effectué de la manière suivante :
Des coupes fraîches de peaux humaines, provenant de 2 donneurs différents, sont traitées pendant 2 heures, à 20°C, par 20 µl de solution tampon (pH 7,4) comprenant éventuellement 10 µg/ml d'ELH et éventuellement 0,1% du composé à tester, éventuellement préalablement mis en solution dans l'éthanol.

Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | % de surface occupée par les fibres élastiques | |
|---|---|---|
| | Peau 1 | Peau 2 |
| Témoin (peau non traitée) | 12,7% | 15,25% |
| Peau traitée avec ELH | 4,85% | 6,85% |
| Peau traitée avec ELH + composé de l'exemple 2 | 13,95% | 11,85% |

On constate donc que le composé selon l'invention génère une protection significative des peaux vis-à-vis de la destruction des fibres élastiques induite par l'élastase.

### Exemple 5

On a évalué l'activité ex vivo du composé de l'exemple 2 sur des peaux humaines en survie traitées par de l'élastase leucocytaire humaine (ELH).

Le test est effectué de la manière suivante :
Des fragments de peau humaine normale provenant de trois donneurs différents sont déposées dans des inserts positionnés dans des puits de culture. On ajoute du milieu de culture supplémenté en antibiotiques dans le fond des puits. Un passage s'effectue par diffusion lente entre les deux compartiments par l'intermédiaire d'une membrane poreuse (taille des pores : 12 µm).
Le milieu de culture est renouvelé tous les trois jours.
On ajoute sur les fragments de peau éventuellement 0,5 µg d'ELH par ml de milieu de culture.
On ajoute également, tous les deux jours, 5 µl du composé à tester, préalablement mis en solution à 0,2% en poids dans l'éthanol.
Les peaux sont maintenues en survie pendant 10 jours à 37°C.

Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | % de surface occupée par les fibres élastiques |
|---|---|
| Témoin (peau non traitée) | 7,4% |
| Peau traitée avec ELH | 5,1% |
| Peau traitée avec ELH + composé de l'exemple 2 | 7,1 % |

On constate donc que le composé selon l'invention génère une protection significative des peaux vis-à-vis de la destruction des fibres élastiques induite par l'élastase.

### Exemple 6

On a évalué l'activité du composé de l'exemple 2 sur des peaux humaines en survie irradiées par des UVA (8 J/cm²).

Le test est effectué de la manière suivante :
Des fragments de peau humaine normale provenant de quatre donneurs différents sont déposées dans des inserts positionnés dans des puits de culture. On ajoute du milieu de culture supplémenté en antibiotiques dans le fond des puits. Un passage s'effectue par diffusion lente entre les deux compartiments par l'intermédiaire d'une membrane poreuse (taille des pores : 12 µm).
Le milieu de culture est renouvelé tous les trois jours.
On ajoute sur les fragments de peau, tous les deux jours, 5 µl d'une solution à 0,2 % du composé à tester en solution dans l'éthanol.
Les peaux sont maintenues en survie pendant 7 jours à 37°C.
Les peaux sont irradiées une seule fois à 8 J/cm² (lampe Vilbert-Lourmat RMX-3W).
Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | Analyse morphométrique des fibres élastiques (derme superficiel) | Analyse morphométrique du collagène (derme superficiel) |
|---|---|---|
| Peau non traitée | 6,75% | 87% |
| Peau traitée par des UVA (8 J/cm²) | 3,9% | 81% |
| Peau traitée par des UVA (8 J/cm²) + composé | 6,8% | 92% |

On constate que le composé selon l'invention a bien une activité vis-à-vis de la dégradation des fibres élastiques dans le derme superficiel de peaux irradiées par des UVA.

Ce composé présente également un effet adéquat sur la protection du collagène.

### Exemple 7 : composition pour application topique

On prépare l'émulsion suivante de façon classique (% en poids) :
- composé de l'Exemple 1 1 %
- rétinol 0,1%
- isostéarate de propylène glycol 13 %
- polyéthylène glycol (8 OE) 5 %
- propylène glycol 3 %
- pentylène glycol 3 %
- stéarate de glycéryle et stéarate de polyéthylène glycol (100 OE) 5 %
- mono-stéarate de sorbitane oxyéthyléné (20 OE) 0,5 %
- alcool cétylique oxyéthyléné (20 OE) oxypropyléné (5 OP) 1 %
- gélifiants 0,5 %
- benzoates d'alkyle en C₁₂₋₁₅ 4 %
- éthanol 3 %
- hydroxyde de sodium 0,12 %
- conservateurs qs
- eau qsp 100 %

### Exemple 8 : crème de soin du visage

On prépare l'émulsion huile-dans-eau suivante de façon classique (% en poids) :
- Composé de l'exemple 2 1%
- Lycopène (sous forme de Lycomato® à 10% de Lycopène dans une Oléorésine de tomate commercialisée par Lycored®) 0,001 %
- Stearate de glycérol 2%
- Polysorbate 60 (Tween 60® vendu par la société ICI) 1 %
- Acide stéarique 1,4%
- Triéthanolamine 0,7%
- Carbomer 0,4%
- Fraction liquide du beurre de karité 12 %
- Perhydrosqualène 12 %
- Antioxydant qs
- Parfum qs
- Conservateur qs
- Eau qsp 100 %

### Exemple 9 : lait pour le visage

On prépare le lait suivant de façon classique (% en poids) :
- Huile de vaseline 7%
- Genistéine 0,2%
- Composé de l'exemple 2 1%
- Monostéarate de glycéryle, stéarate de polyéthylène glycol (100 OE) 3%
- Polymère carboxyvinylique 0,4 %
- Alcool stéarylique 0,7 %
- Protéines de soja 3%
- NaOH 0,4 %
- Conservateur qs
- Eau qsp 100 %

### Exemple 10 : lotion capillaire

On prépare la lotion suivante de façon classique (% en poids) :
- composé de l'Exemple 1 1 %
- Rétinol 0,01 %
- propylène glycol 23 %
- éthanol 55%
- eau qsp 100 %

On peut appliquer cette lotion sur le scalp des individus alopéciques, pour prévenir les effets des UV, avant et/ou après exposition au soleil.

### Exemple 11 : lotion antichute

On prépare la lotion suivante de façon classique (% en poids) :
- composé de l'Exemple 2 1 %
- Lycopène (sous forme de Lycomato® à 10% de Lycopène dans une Oléorésine de tomate commercialisée par Lycored®) 0,0001 %
- propylène glycol 23 %
- éthanol 55 %
- Aminexil 1,5 %
- eau qsp 100 %

On peut appliquer cette lotion antichute sur le scalp des individus alopéciques.

## Revendications

1. Composition cosmétique ou dermatologique comprenant une association entre un composé inhibiteur de l'élastase de la famille des N-Acylaminoamides et au moins un composé inhibiteur de métalloprotéinase,
**caractérisée en ce que** le composé inhibiteur de la famille des N-Acylaminoamides est un composé de formule (I) : 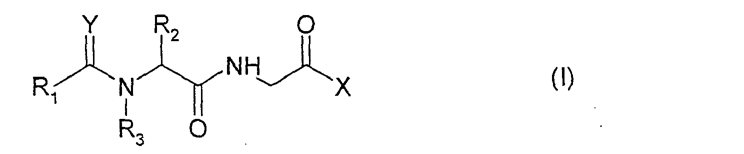 dans laquelle :
- y représente l'oxygène
- le radical R1 représente un radical méthyle, éthyle, propyle ou isopropyle, éventuellement substitué par un groupement -OH ou -P(O)-(OR)₂ avec R représentant méthyle, éthyle, propyle ou isopropyle; et/ou
- le radical R2 représente un radical méthyle, éthyle, propyle, isopropyle, n-butyle, ter-butyle ou isobutyle; et/ou
- le radical R3 représente un groupement choisi parmi l'une des formules suivantes : 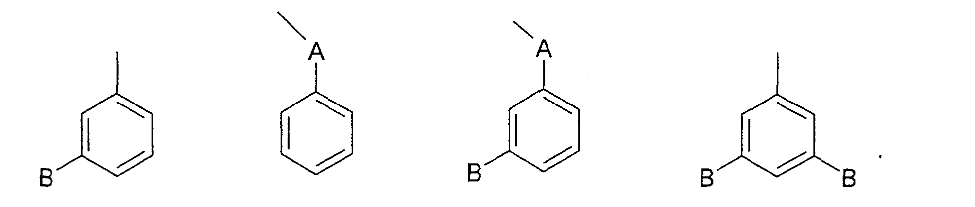
dans lesquelles le radical divalent A est un méthylène, un éthylène, un propylène et/ou le radical B est un radical méthyle, éthyle, propyle ou isopropyle, substitué par un ou plusieurs halogènes, notamment chlore, brome, iode ou fluor, et préférentiellement totalement halogéné (perhalogéné), tel que perfluoré, notamment le radical perfluorométhyle (-CF3).
- le radical X représente un radical choisi parmi -OH, -OCH₃, -OC₂H₅, -O-C₃H₇ ou -OC₄H₉, ou -OC₄H₉, ses sels d'acide minéral ou organique, ses isomères optiques, sous forme isolée ou un mélange racémique.

2. Composition selon la revendication 1, dans laquelle le composé de formule (I) est choisi parmi les composés suivants :
- l'acide {2-[acétyl-(3-trifluorométhyl-phényl)-amino]-3-méthyl-butyrylamino} acétique,
- le {2-[acétyl-(3-trifluorométhyl-phényl)-amino]-3-méthyl-butyrylamino; acétate d'éthyle,
- l'acide [2-(acétyl-benzyl-amino)-3-méthyl-butyrylamino] acétique,
- le [2-(acétyl-benzyl-amino)-3-méthyl-butyrylamino] acétate d'éthyle,
- le (2-{benzyl-[(diethoxy-phosphoryl)-acétyl]-amino}-3-méthyl-butyrylamino) acétate d'éthyle.

3. Composition selon l'une des revendications précédentes, dans laquelle le composé inhibiteur de l'élastase de la famille des N-Acylaminoamides est présent en une quantité comprise entre 0,00001 et 20% en poids par rapport au poids total de la composition, et préférentiellement entre 0,0001 et 5% en poids.

4. Composition selon l'une des revendications précédentes, **caractérisée en ce que** l'inhibiteur de métalloprotéinase est choisi parmi un inhibiteur de l'activité et/ou de l'expression et/ou de la synthèse de métalloprotéinase choisie parmi les MMP-1, MMP-2, MMP-3, MMP-7, MMP-9, MMP-11, MMP-12, MMP-13, MMP-14, MMP-15, MMP-16 et MMP-17.

5. Composition selon l'une des revendications 1 à 3, **caractérisée en ce que** l'inhibiteur de métalloprotéinase est un inhibiteur tissulaire de métalloprotéinases (TIMP), tel que les peptides connus sous les appellations TIMP-1, TIMP-2, TIMP-3 et TIMP-4.

6. Composition selon l'une des revendications 1 à 3, **caractérisée en ce que** l'inhibiteur de métalloprotéinase est d'origine naturelle ou synthétique.

7. Composition selon l'une des revendications 1 à 3, **caractérisée en ce que** l'inhibiteur de métalloprotéinase est choisi parmi l'adapalène ou des peptides analogues et/ou des dérivés du Batimastat, du Marimastat et/ou des caroténoïdes et/ou des filtres solaires et/ou de la vitamine C et/ou des isoflavones.

8. Composition selon l'une des revendications 1 à 3, **caractérisée en ce que** l'inhibiteur de métalloprotéinase est choisi parmi le rétinol et ses dérivés ou l'acide rétinoïque et ses dérivés ou la genistéine ou la daidzéine ou le lycopène.

9. Composition selon l'un des revendications précédentes, **caractérisée en ce que** l'inhibiteur de métalloprotéinase représente de préférence de 10⁻¹² à 5%, et de préférence de 10⁻¹⁰ à 2%, du poids total de la composition

10. Composition selon l'une des revendications précédentes, se présentant sous la forme d'une composition cosmétique ou dermatologique destinée au soin et/ou au traitement des zones ulcérées ou ayant subi un stress ou microstress cutané, notamment généré par une exposition aux UV et/ou la mise en contact avec un produit irritant.

11. Composition selon l'une des revendications 1 à 9, se présentant sous la forme :
- d'un produit de soin, de traitement, de nettoyage ou de protection, de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées;
- d'une composition de protection solaire, de bronzage artificiel (autobronzant) ou de soin après-soleil;
- d'une composition capillaire, et notamment une crème ou un gel protecteur solaire; une composition de soin du cuir chevelu, notamment anti-chute ou repousse des cheveux;
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres;
- d'un produit d'hygiène buccale tel qu'une pâte dentifrice ou une lotion de rinçage buccal.

12. Composition selon l'une des revendications 1 à 9, se présentant sous la forme d'une composition de soin de la peau du visage, de type anti-rides ou anti-age, ou d'une composition de protection solaire ou après-soleil.

13. Procédé de traitement cosmétique de la peau du corps ou du visage, y compris le cuir chevelu, dans lequel on applique sur la peau une composition cosmétique telle que définie dans l'une des revendications 1 à 12.

## Claims

1. A cosmetic or dermatological composition comprising a combination between an elastase inhibitor compound of the N-acylaminoamides and at least a metalloproteinase inhibitor, **characterized in that** the inhibitor compound of the N-acylaminoamides is a compound of the formula (I) : 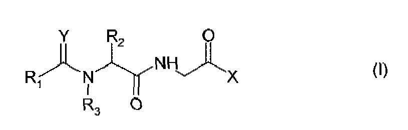 wherein :
- Y represents oxygene
- the R₁ radical represents a methyl, ethyl, propyl or isopropyl radical, optionally substituted by a -OH or -P(O)-(OR)₂ group with R representing a methyl, ethyl, propyl or isopropyl group; and/or
- the R₂ radical represents a methyl, ethyl, propyl, isopropyl, n-butyl, ter-butyl or isobutyl radical; and/or
- the R₃ radical represents a group selected amongst one of the following formulae: 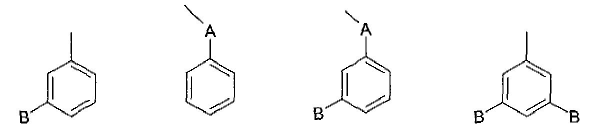
wherein the divalent A radical is a methylene, ethylene, propylene group and/or the B radical is a methyl, ethyl, propyl or isopropyl radical, substituted by one or more halogens, in particular, chlorine, bromine, iodine or fluorine, and preferably wholly halogenated (perhalogenated), such as perfluorinated, more particularly the (CF₃) perfluoromethyl radical,
- the X radical represents a radical selected amongst -OH, -OCH₃, -OC₂H₅, -O-C₃H₇ or -OC₄H₉, the inorganic or organic acid salts thereof, the optical isomers thereof either isolated or as a racemic mixture.

2. A composition according to claim 1, wherein the compound of formula (I) is selected amongst the following compounds:
- {2-[acetyl-(3-trifluoromethyl-phenyl)-amino]-3-methyl-butyrylamino} acetic acid,
- {2-[acetyl-(3-trifluoromethyl-phenyl)-amino]-3-methyl-butyrylamino} ethyl acetate,
- [2-(acetyl-benzyl-amino)-3-methyl-butyrylamino] acetic acid,
- [2-(acetyl-benzyl-amino)-3-methyl-butyrylamino] ethyl acetate, and
- (2-{benzyl-[(diethoxy-phosphoryl)-acetyl]-amino}-3-methyl-butyryl-amino] ethyl acetate.

3. A composition according to one of the preceding claims, wherein the elastase inhibitor compound of the N-acylaminoamides is in an amount ranging from 0.00001 to 20% by weight based on the total weight of the composition, preferably from 0.0001 to 5% by weight.

4. A composition according to one of the preceding claims, **characterized in that** the metalloproteinase inhibitor is selected amongst an inhibitor of the activity and/or the expression and/or the synthesis of metalloproteinase selected amongst MMP-1, MMP-2, MMP-3, MMP-7, MMP-9, MMP-11, MMP-12, MMP-13, MMP-14, MMP-15, MMP-16 and MMP-17.

5. A composition according to one of claims 1 to 3, **characterized in that** the metalloproteinase inhibitor is a tissue inhibitor of metalloproteinases (TIMP) such as the peptides known under the names TIMP-1, TIMP-2, TIMP-3 and TIMP-4.

6. A composition according to one of claims 1 to 3, **characterized in that** the metalloproteinase inhibitor is from natural or synthetic origin.

7. A composition according to one of claims 1 to 3, **characterized in that** the metalloproteinase inhibitor is selected amongst adapalene or analogous peptides and/or derivatives of Batimastat, Marimastat and/or carotenoids and/or sunscreens and/or vitamin C and/or isoflavones.

8. A composition according to one of claims 1 to 3, **characterized in that** the metalloproteinase inhibitor is selected amongst retinol and the derivatives thereof or retinoic acid and the derivatives thereof or genistein or daidzein or lycopene.

9. A composition according to one of the preceding claims, **characterized in that** the metalloproteinase inhibitor preferably represents from 10⁻¹² to 5%, more preferably from 10⁻¹⁰ to 2% of the total weight of the composition.

10. A composition according to one of the preceding claims, having the form of a cosmetic or dermatological composition, designed for caring and/or treating ulcerated areas or having been subjected to a cutaneous stress or microstress, more particularly, generated by an exposure to the UV and/or the contact with an irritating product.

11. A composition according to one of claims 1 to 9, exhibiting the form of:
- a care, treatment, cleaning or protection product, of the face or body skin, including the scalp, such as a (day, night, hydrating) care composition for the face or the body; an anti-wrinkle or anti-ageing composition for the face; a mating composition for the face; a composition for the irritated skins;
- a sun protective, artificial sun tanning (self-tanning) or after-sun care composition;
- a capillary composition, more particularly, a sun protective cream or gel; a scalp care composition, including a hair restoring or hair growth composition;
- a face skin, body or lip make-up product, such as a foundation cream, a tinted cream, a cheek or eye-lid make-up product, a free or compact powder, an anti eye-ring stick, a concealing stick, a lipstick, a lip care product; and
- a mouth hygiene product, such as a tooth-paste or a mouthwash lotion.

12. A composition according to one of claims 1 to 9, having the form of a face skin care composition, of the anti-wrinkle or anti-ageing type, or a sun protective or after-sun composition.

13. A method for cosmetically treating the body or the face skin, including the scalp, wherein a cosmetic composition such as defined in one of claims 1 to 12 is applied onto the skin.

## Patentansprüche

1. Kosmetische oder dermatologische Zusammensetzung, umfassend eine Assoziation zwischen einer Inhibitorverbindung der Elastase der Familie der N-Acylaminoamide und wenigstens einer Inhibitorverbindung der Metalloproteinase, **dadurch gekennzeichnet, dass** die Inhibitorverbindung der Familie der N-Acylaminoamide eine Verbindung der Formel (I) ist: 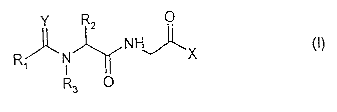 in der
- Y für Sauerstoff steht,
- der Rest R1 einen Methyl-, Ethyl-, Propyl- oder Isopropylrest darstellt, der gegebenenfalls durch eine Gruppierung -OH oder -P(O)-(OR)₂, wobei R Methyl, Ethyl, Propyl oder Isopropyl darstellt, substituiert ist; und/oder
- der Rest R2 einen Methyl-, Ethyl-, Propyl-, Isopropyl-, n-Butyl-, tert-Butyl- oder Isobutylrest darstellt; und/oder
- der Rest R3 eine Gruppierung darstellt, die unter den folgenden Formeln ausgewählt ist: 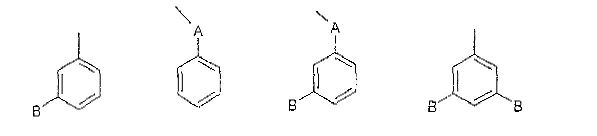 in denen der zweiwertige Rest A ein Methylen, ein Ethylen, ein Propylen ist und/oder der Rest B ein Methyl-, Ethyl-, Propyl- oder Isopropylrest ist, der mit einem oder mehreren Halogen (en) substituiert ist, insbesondere mit Chlor, Brom, Iod oder Fluor, und vorzugsweise vollständig halogeniert ist (perhalogeniert ist), wie perfluoriert ist, insbesondere der Perfluormethylrest (-CF₃) ist;
- der Rest X einen Rest darstellt, ausgewählt aus -OH, -OCH₃, -OC₂H₅, -O-C₃H₇ oder -OC₄H₉,
ihre Salze mit Mineralsäuren oder organischen Säuren, ihre optischen Isomere in isolierter Form oder als racemisches Gemisch ist.

2. Zusammensetzung nach Anspruch 1, wobei die Verbindung der Formel (I) aus den folgenden Verbindungen ausgewählt ist:
- {2-[Acetyl-(3-trifluormethylphenyl)-amino]-3-methyl-butyrylamino}essigsäure,
- {2-[Acetyl-(3-trifluormethylphenyl)-amino]-3-methyl-butyrylamino}essigsäureethylester,
- [2-(Acetylbenzylamino)-3-methylbutyrylamino]essigsäure,
- [2-(Acetylbenzylamino)-3-methylbutyrylamino]essigsäureethylester,
- (2-{Benzyl-[(diethoxyphosphoryl)-acetyl]-amino}-3-methylbutyrylamino)essigsäureethylester.

3. Zusammensetzung nach einem der vorangehenden Ansprüche, wobei die Inhibitorverbindung der Elastase der Familie der N-Acylaminoamide in einer Menge zwischen 0,00001 und 20 Gew.-%, bezogen auf das Gesamtgewicht der Zusammensetzung, und vorzugsweise zwischen 0,0001 und 5 Gew-%, vorliegt.

4. Zusammensetzung nach einem der vorangehenden Ansprüche, **dadurch gekennzeichnet, dass** der Inhibitor der Metalloproteinase ausgewählt ist unter einem Inhibitor der Aktivität und/oder der Expression und/oder der Synthese der Metalloproteinase, ausgewählt unter MMP-1, MMP-2, MMP-3, MMP-7, MMP-9, MMP-11, MMP-12, MMP-13, MMP-14, MMP-15, MMP-16 und MMP-17.

5. Zusammensetzung nach einem der Ansprüche 1 bis 3, **dadurch gekennzeichnet, dass** der Inhibitor der Metalloproteinase ein Gewebeinhibitor der Metalloproteinasen (TIMP) wie die Peptide, die unter den Bezeichnungen TIMP-1, TIMP-2, TIMP-3 und TIMP-4 bekannt sind, ist.

6. Zusammensetzung nach einem der Ansprüche 1 bis 3, **dadurch gekennzeichnet, dass** der Inhibitor der Metalloproteinase natürlicher oder synthetischer Herkunft ist.

7. Zusammensetzung nach einem der Ansprüche 1 bis 3, **dadurch gekennzeichnet, dass** der Inhibitor der Metalloproteinase ausgewählt ist aus Adapalen oder analogen Peptiden und/oder Derivaten von Batimastat, Marimastat und/oder Carotinoiden und/oder Sonnenfiltern und/oder Vitamin C und/oder Isoflavonen.

8. Zusammensetzung nach einem der Ansprüche 1 bis 3, **dadurch gekennzeichnet, dass** der Inhibitor der Metalloproteinase ausgewählt ist unter Retinol und seinen Derivaten oder Retinoesäure und ihren Derivaten oder Genistein oder Daidzein oder Lycopen.

9. Zusammensetzung nach einem der vorangehenden Ansprüche, **dadurch gekennzeichnet, dass** der Inhibitor der Metalloproteinase vorzugsweise 10⁻¹² bis 5% und bevorzugter 10⁻¹⁰ bis 2% des Gesamtgewichts der Zusammensetzung darstellt.

10. Zusammensetzung nach einem der vorangehenden Ansprüche, die sich in Form einer kosmetischen oder dermatologischen Zusammensetzung präsentiert, die zur Pflege und/oder zur Behandlung von Bereichen bestimmt ist, welche ulzeriert sind oder einem kutanen Stress oder kutanem Mikrostress unterworfen waren, der insbesondere durch eine Exposition gegenüber UV und/oder dem Inkontaktbringen mit einem reizenden Produkt erfolgte.

11. Zusammensetzung nach einem der Ansprüche 1 bis 9, die sich in der Form:
eines Produktes zur Pflege, Behandlung, Reinigung oder zum Schutz der Haut, des Gesichts oder des Körpers, hier eingeschlossen die Kopfhaut, wie zum Beispiel eine Zusammensetzung zur Pflege (für Tag, Nacht, hydratisierend) des Gesichts oder des Körpers; eine Anti-Falten-Zusammensetzung oder eine Anti-Age-Zusammensetzung für das Gesicht; eine mattierende Zusammensetzung für das Gesicht; eine Zusammensetzung für die irritierte Haut;
einer Sonnenschutzzusammensetzung, einer Zusammensetzung zur künstlichen Bräunung (Selbstbräunungsmittel) oder einer Zusammensetzung zur Pflege nach dem Sonnen;
- einer Haar-Zusammensetzung und insbesondere einer Sonnenschutzcreme oder eines Sonnenschutzgels; einer Zusammensetzung zur Pflege der Kopfhaut, insbesondere gegen Schuppen oder für das Wiedersprießen der Haare;
- eines Make-up-Produktes für die Haut des Gesichts, des Körpers oder der Lippen, zum Beispiel Grundierung, eine getönte Creme, Schminke für Wangen oder Augenlider oder lockeres oder kompaktes Puder, ein Stift gegen Augenringe, ein Deckstift, ein Lippenstift, eine Lippenpflege;
- eines buccalen Hygieneproduktes, wie zum Beispiel Zahnpasta oder Mundspülung,
präsentiert.

12. Zusammensetzung nach einem der Ansprüche 1 bis 9, die sich in Form einer Zusammensetzung zur Pflege der Haut des Gesichts, des Anti-Falten-Typs oder Anti-Age-Typs oder in Form einer Sonnenschutzzusammensetzung oder einer Zusanunensetzung zur Anwendung nach dem Sonnen präsentiert.

13. Verfahren zur kosmetischen Behandlung der Haut des Körpers oder des Gesichts, hier eingeschlossen die Kopfhaut, bei dem man eine kosmetische Zusammensetzung, wie sie in einem der Ansprüche 1 bis 12 definiert ist, auf die Haut anwendet.
